(19) Europäisches Patentamt
European Patent Office
Office européen des brevets

(11) Veröffentlichungsnummer: **0 000 359**
**B1**

(12) # EUROPÄISCHE PATENTSCHRIFT

(45) Veröffentlichungstag der Patentschrift: **21.10.81**

(21) Anmeldenummer: **78100281.1**

(22) Anmeldetag: **30.06.78**

(51) Int. Cl.³: **C 07 C 147/00,**
A 01 N 41/02

(54) **Phenoxy-phenylsulfinyl- und -sulfonyl-alkancarbonsäure-Derivate, Verfahren zu deren Herstellung und deren Verwendung als Herbizide und als Pflanzenwachstumsregulierungsmittel.**

(30) Priorität: **07.07.77 CH 8426/77**

(43) Veröffentlichungstag der Anmeldung:
**24.01.79 Patentblatt 79/2**

(45) Bekanntmachung des Hinweises auf die Patenterteilung:
**21.10.81 Patentblatt 81/42**

(84) Benannte Vertragsstaaten:
**BE CH DE FR GB NL**

(56) Entgegenhaltungen:
**DE - A - 2 333 848**
**DE - A - 2 223 894**
**DE - A - 2 130 919**
**FR - A - 2 325 638**
**FR - A - 2 285 861**
**CH - A - 424 761**

(73) Patentinhaber: **CIBA-GEIGY AG**
**Patentabteilung Postfach**
**CH-4002 Basel (CH)**

(72) Erfinder: **Dürr, Dieter, Dr.**
**Brändelistalweg 16**
**CH-4103 Bottmingen (CH)**
Erfinder: **Rohr, Otto, Dr.**
**Kilbertweg 19**
**CH-4106 Therwil (CH)**
Erfinder: **Pissiotas, Georg, Dr.**
**Breslauerstrasse 8**
**D-7850 Lörrach (DE)**
Erfinder: **Böhner, Beat, Dr.**
**Hügelweg 3**
**CH-4102 Binningen (CH)**

**EP 0 000 359 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Phenoxy-phenylsulfinyl- und -sulfonyl-alkancarbonsäure-Derivate, Verfahren zu deren Herstellung und deren Verwendung als Herbizide und als Pflanzenwachstumsregulierungsmittel

Die vorliegende Erfindung betrifft neue Phenoxy-phenyl-sulfinyl- und -sulfonyl-alkancarbonsäure-Derivate, welche herbizide und das Pflanzenwachstum regulierende Wirkung zeigen, Verfahren zur Herstellung dieser Derivate, herbizide und das Pflanzenwachstum regulierende Mittel, welche diese Derivate als Wirkstoffe enthalten sowie die Verwendung der neuen Phenoxy-phenyl-sulfinyl- und -sulfonyl-alkancarbonsäurederivate oder sie enthaltender Mittel als Herbizide und/oder zur Regulierung des Pflanzenwachstums.

Die neuen Phenoxy-phenyl-sulfinyl- und -sulfonyl-alkancarbonsäure-Derivate entsprechen der Formel I

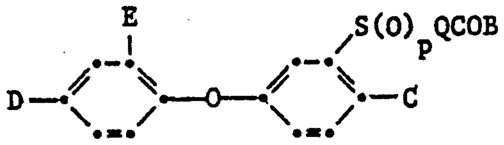

(I)

worin

B einen Rest $—OR_1$, $—SR_2$ oder $—NR_3R_4$,
C Wasserstoff, Halogen Nitro, $C_1$—$C_4$ Alkyl oder $C_1$—$C_4$ Alkoxy,
D Halogen, Cyan, Nitro oder Trifluormethyl,
E Wasserstoff, Halogen, Cyan oder Nitro,
p die Zahl 1 oder 2,
Q eine unverzweigte oder verzweigte $C_1$—$C_4$ Alkylenbrücke,
$R_1$ Wasserstoff oder das Kation eines Alkali- oder Erdalkalimetalles, einen $C_1$—$C_4$ Alkylammonio-Rest, der im Alkylteil durch Hydroxyl, Cyan oder $C_1$—$C_4$ Alkoxy substituiert sein kann,
— $C_1$—$C_{18}$ Alkyl, unsubstituiert oder substituiert durch Halogen, Nitro, Cyan, $C_1$—$C_8$ Alkoxy, Bis ($C_1$—$C_4$ alkyl)-amino,
— $C_3$—$C_{18}$ Alkenyl, gegebenenfalls Halogen substituiert,
— $C_3$—$C_8$ Alkinyl
— $C_3$—$C_{12}$ Cycloalkyl,
— Phenyl oder Benzyl, unsubstituiert oder substituiert durch Halogen, $C_1$—$C_4$ Alkyl, $C_1$—$C_4$ Alkoxy, $C_1$—$C_4$ Alkylthio, Nitro, Cyan oder Trifluormethyl;
$R_2$ $C_1$—$C_{18}$ Alkyl,
— Phenyl- oder Benzyl, unsubstituiert oder substituiert durch Halogen, $C_1$—$C_4$ Alkyl, $C_1$—$C_4$ Alkoxy, $C_1$—$C_4$ Alkylthio, Nitro, Cyan oder Trifluormethyl;
$R_3$ und $R_4$ Wasserstoff, $C_1$—$C_4$ Alkyl oder 2-Methoxyethyl bedeuten.

Die Alkyl- und Alkenylreste in dieser Definition können verzweigt oder unverzweigt sein.

Die vorliegenden Phenoxy-phenylsulfinyl- und -sulfonyl-alkancarbonsäurederivate sind neue Verbindungen. Phenoxyphenylthio-alkancarbonsäurederivate mit ähnlicher Struktur, welche als Ausgangsstoffe zur Herstellung vorliegender Verbindungen benutzt werden können, sind bekannt. Die FR—PS 285 861 beschreibt Verbindungen, welche pharmakologische Wirkung zeigen, die DOS 2 223 894 solche mit herbizider Wirkung. Ferner sind herbizid wirkende Diphenyläther, mit Alkyl- und Alkenylsulfinyl- oder -sulfonylresten in den DT-OS 2 130 919 und 2 333 848 beschrieben worden. Die Verbindungen der Formel I besitzen eine herbizide Wirkung, die nicht gegen jede Pflanzenart gleich stark wirkt. Dadurch entstehen wertvolle Selektivitäten, die es ermöglichen, spezifisch Unkräuter in Kulturpflanzungen zu bekämpfen, ohne dabei die Kulturpflanzen merklich zu schädigen. Die beste Wirkung wird im Nachauflaufverfahren in Getreide- und Reiskulturen gegen breitblättrige Unkräuter erreicht.

Die Wirkstoffe der Formel I besitzen ferner günstige wachstumsregulierende Effekte (Wuchshemmung). Insbesondere hemmen sie das Waschstum von dicotylen Pflanzen. Beispiele für die nutzbringende Anwendung der erfindungsgemässen Verbindung sind z.B.

— die Reduktion des veregativen Wachstums bei Soja und ähnlichen Leguminosen, was zu einer Ertragssteigerung dieser Kultur führt;
— die Hemmung des unerwünschten Wachstums von Geiztrieben bie Tabak, dessen Haupttrieb man geschnitten hat, was der Ausbildung grösserer und schönerer Blätter zugute kommt;
— die Hemmung des Wachstums von Gras und dikotyledonen Pflanzen, wie Obstbäume, Zierbäume, Gebüsche und Hecken, zwecks Einsparung an Schnittarbeit.

Die Verbindungen vorliegender Erfindung sind wenig giftig für Warmblüter und deren Applikation wirft keine Probleme auf. Die Aufwandmenge liegt zwischen 0.1 und 5 kg pro Hektar.

Die Herstellung der neuen Verbindungen der Formel I erfolgt nach an sich bekannten Methoden und kann aus dem folgenden Formelschema ersehen werden:

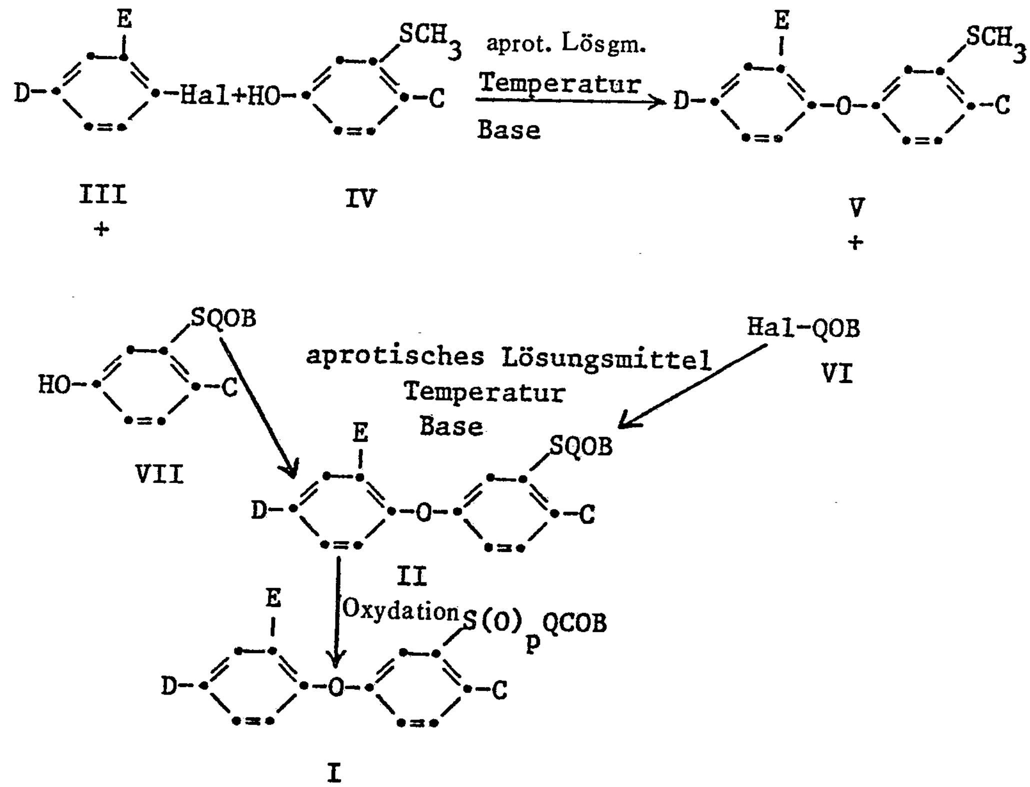


III + IV → V + VI; VII → II → I

In deisen Formeln haben B, C, D, E, Q und p die unter Formel I angegebene Bedeutung und Hal bedeutet ein Halogenatom, vorzugsweise Chlor oder Brom.

Man geht beispielsweise von einem gegebenenfalls substituierten Halogenbenzol der Formel III aus und kondensiert dieses mit einem gegebenenfalls weiter substituierten Methylthio-phenol der Formel IV in Gegenwart eines säurebindenden Mittels in einem aprotischen Lösungsmittel bei erhöhter Temperatur zur Phenoxy-methylthio-Verbindung der Formel V. Diese kann dann weiter mit einem $\alpha$-Halogenalkancarbonsäurederivat der Formel VI bei erhöhter Temperatur umgesetzt werden, worauf man zur Phenoxy-phenyl-thio-alkancarbonsäure der Formel II gelangt. Die Herstellung solcher Phenoxy-phenylthioalkancarbonsäure-Derivate ist unter anderem auch in der DOS 2 223 894 beschrieben.

Ein weiterer Weg um zu diesen Phenoxy-phenylthio-alkancarbonsäure-Derivaten der Formel II zu gelangen, geht ebenfalls von den gegebenenfalls substituierten Halogenbenzolen der Formel III aus, welche man z.B. mit einer gegebenenfalls im Ring weiter substituierten Hydroxyphenylthio-alkancarbonsäure der Formel VII kondensiert, in Gegenwart eines säurebindenden Mittels, wie z.B. einer anorganischen Base, in einem aprotischen Lösungsmittel bei erhöhter Temperatur.

Das erfindungsgemässe Verfahren zur Herstellung der neuen Phenoxyphenylsulfinyl- und -sulfonyl-alkancarbonsäure-Derivaten ist dadurch gekennzeichnet, dass man in an sich bekannter Weise ein Phenoxy-phenylthio-alkancarbonsäure-Derivat der Formel II (siehe Formelschema), worin B, C, D, E und Q die unter Formel I gegebene Bedeutung haben, solange mit einem oxydierenden Mittel behandelt, bis die Sulfid-Brücke zur Sulfinyl-(—SO) oder Sulfonyl-Brücke (—$SO_2$) oxydiert ist.

Geeignete oxydierende Mittel für diese Reaktion sind beispielsweise wasserstoffperoxyd, Sauerstoff oder gegebenenfalls auch Luft, die durch das Reaktionsgemisch geblasen wird. Ferner kann man das Phenoxy-phenylthio-alkancarbonsäure-Derivat in verdünnte Natronlauge geben und dann langsam Chlor durch das Reaktionsgemisch blasen.

Ferner kann man die Verbindungen der Formel I auch herstellen, indem man ein entsprechend substituiertes Diphenyl-äther-sulfochlorid mit Natriumsulfit zur Sulfinsäure reduziert und diese, respektive ein Salz derselben, dann mit einem Rest Hal—Q—COB, worin B, Q und Hal die unter Formel I gegebene Bedeutung haben, umsetzt.

Die nachfolgenden Beispiele veranschaulichen die Herstellung der Wirkstoffe der Formel I. Weitere in entsprechender Weise hergestellte Wirkstoffe sind in den anschliessenden Tabellen aufgeführt. Temperaturangaben beziehen sich jeweils auf Celsius-Grade. Druckangaben werden in Millibar (mbar) gegeben.

In einer weiteren Tabelle sind als Zwischenprodukte erhaltene Phenoxy-phenylthio-alkancarbonsäuren der Formel II aufgeführt.

Beispiel 1

3-(2',4'-Dichlorphenoxy)-6-jod-phenylsulfinyl-essigsäure-methylester

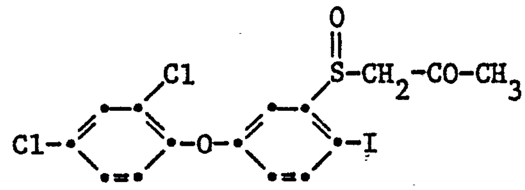

a) 12 g 4-Chlor-2-methylmerkapto-nitrobenzol werden in 50 ml Dimethylsulfoxyd mit 10 g 2,4-Dichlorphenol und 2,5 g Natriumhydroxyd auf 160° erhitzt. Nach zwei Stunden giesst man den Ansatz auf Eis und extrahiert mit Essigester. Die organische Lösung wird eingedampft und der Rückstand aus Toluol/Hexan umkristallisiert. Man erhält 16 g 2,4-Dichlor-3'-methylmerkapto-4''-nitrodiphenyläther vom Schmelzpunkt 120—122°. Die katalytische Reduktion in Dioxan mit Raney-Nickel ergibt daraus in nahezu quantitativer Ausbeute 2,4-Dichlor-3'-methylmerkapto-4'-aminodiphenyläther. Smp. 40—42°.

b) 65 g dieser Verbindung werden mit 300 ml Wasser und 144 ml konz. Schwefelsäure vermischt und auf 0° gekühlt. Durch Umsatz mit einer 5%igen Natriumnitritlösung bei 0—5° bereitet man eine Diazoniumlösung, in die nach einer Stunde bei 0° eine Lösung von 39,5 g Kaliumjodid in 270 ml Wasser eingetropft wird. Es bildet sich ein klebriger Niederschlag, der nach 2 Stunden durch Zugabe von Methylenchlorid gelöst wird. Die organische Phase wird abgetrennt und eingedampft. Der Rückstand wird mehrnals mit warmem Hexan extrahiert und die vereinigten Hexanextrakte eingeengt und abgekühlt. Durch Kristallisation erhält man 2,4-Dichlor-3'-methylmerkapto-4-jod-diphenyläther.

c) 10 g 2,4-Dichlor-3'-methylmerkapto-4'-jod-diphenyläther wurden mit 20 ml Bromessigsäuremethylester 10 Stunden am Rückfluss erhitzt, die Reinigung erfolgte chromatographisch an einer Keiselsäuresäule mit Cyclohexan als Laufmittel. Man erhält nach Verdampfen des Lösungsmittels 7 g 3-(2',4'-Dichlorphenoxy)-6-jod-phenylthio-essigsäuremethylester mit dem Brechungsindex $N_D^{24}$ 1.6528.

d) 5,4 g dieser Verbindung werden in 100 ml Methylenchlorid langsam mit 2,5 g 86%iger 3-Chlorperbenzoesäure versetzt. Nach zwei Stunden wird mit verdünnter Natronlauge extrahiert, die organische Phase über Natriumsulfat getrocknet, eingedampft und der Rückstand aus Toluol/Hexan umkristallisiert. Man erhält 3,5 g der Titelverbindung. Smp. 120—122°.

Beispiel 2

$\alpha$-[3-(2'.4'-dichlorphenoxy)-6-chlor-phenyl-sulfonyl]-propionsäuremethylester.

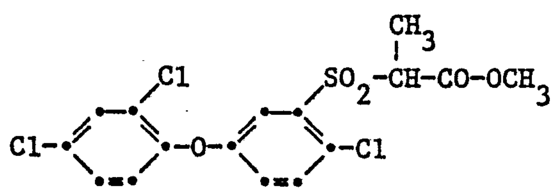

a) Eine Lösung von 400 g Di-(2',4'-dichlorphenoxy)-nitrobenzol, 64 g 85%igem Kaliumhydroxyd und 100 ml Dioxan wird mit 45 g Methylmerkaptan bei 50—70° versetzt. Nach einer Stunde wird das Lösungsmittel am Vakuum abgezogen und der Rückstand mit Toluol aufgenommen. Die durch Waschen der Lösung mit verdünnter Natronlauge vom enstandenen 2,4-Dichlorphenol befreite Lösung wird zu einer siedenden Mischung von 500 g Eisenpulver, 500 g Aethanol und 50 ml konz. Salzsäure getropft. Nach 15 Stunden Rückfluss wird abgekühlt, alkalisch gestellt mit 30%iger Natronlauge, vom Eisenschlamm abgesaugt und der Filterkuchen mit Toluol gewaschen. Die organische Phase des Filtrats wird eingedampft und abdestilliert. Man erhält 192,5 g 4-(2',4'-Dichlorphenoxy-2-methylmerkapto-anilin. Sdp. 170°/0,1 mbar.

b) 180 g der Verbindung Beispiel 3a werden in 1,5 Liter Eisessig gelöst und mit 153 ml konz. Salzsäure versetzt. Durch Eintropfen einer Lösung von 41,5 g Natriumnitrit in 100 ml Wasser bei 5° wird eine Diazolösung hergestellt, die nach dem Zerstören des Nitritüberschusses mit Sulfaminsäure bei 75—90° in 2 Liter einer 18%igen Salzsäure und 118 g Kupfer(I)chlorid getropft wird. Nach 1 Stunde extrahiert man mit Toluol, dampft die organische Phase ein und destilliert den Rückstand. Man erhält 172 g 3-(2'-4'-Dichlorphenoxy)-6-chlor-methylmerkaptobenzol, Sdp. 158°/0,05 mbar.

c) 140 g der Verbindung aus Beispiel 3b wurden mit 105 ml 2-Brom-propionsäuremethylester und einem Kristall Kaliumjodid 16 Stunden am Rückfluss gekocht. Die anschliessende Destillation ergibt 150 g $\alpha$-[3(2',4'-Dichlorphenoxy)-phenol-6-chlorphenyl-thio]-propionsäuremethylester. Sdp. 186°/0,09 mbar.

d) Die behandlung von 7 g dieser Verbindung mit 8,2 g 3-Chlorperoxy-benzoesäure in Methylenchlorid analog Beispiel 1 d ergibt 6 g der Titelverbindung in Form eines Oels vom Brechungsindex $n_D^{24}$ = 1,5890.

Beispiel 3

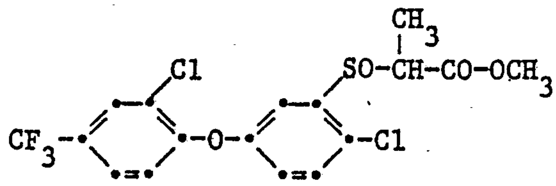

$\alpha$-[3(2′-Chlor-4′-trifluormethylphenoxy)-6-chlor-phenylsulfinyl]-propionsäuremethylester.

a) 241 g 2-Chlor-5-methoxy-anilin werden unter starkem Rühren zu 310 ml Wasser und 385 ml konz. Salzsäure gegeben, gefolgt von 750 g Eis. Man kühlt die Mischung auf —10° und gibt auf einmal eine Lösung von 111 g Natriumnitrit in 150 ml Wasser dazu, dabei steigt die Temperatur auf 10° an. Nach 30 Minuten wird der Nitritüberschuss mit Sulfaminsäure entfernt und die Lösung durch Filtration geklärt. Diese Diazolösung wird bei 55—60° zu einer Lösung von 300 ml Wasser, 800 ml Toluol, 225 g Kaliummethylxanthogenat, 85 g Natriumbicarbonat und 75 ml 30%iger Natronlauge getropft. Nach beendeter, sehr lebhafter Gasentwicklung trennt man die organische Phase ab. Diese wird mit Wasser gewaschen und nach dem Trocknen über Natriumsulfat in 100 ml Triäthylamin bei 70° getropft. 2 Stunden Rückfluss, dann abkühlen und Zugabe von 200 ml 30%iger Natronlauge. Die organische Phase wird abgetrennt und destilliert. Man erhält 160 g 4-chlor-3-methyl-merkaptoanisol. Sdp. 138—145°/11 mbar.

152,2 g dieser Verbindung werden analog J.Am.Chem.Soc. 79, 720 zu 110 g 4-Chlor-3-methyl-merkaptophenol verseift. Sdp. 117—121°/0,03 mbar.

b) 50 g 4-Chlor-3-methylmerkaptophenol, 62 g 3,4-Dichlorbenzotrifluorid und 12 g Natriumhydroxyd werden in 150 ml Dimethylsulfoxyd 6 Stunden bei 142° verrührt, dann giesst man das Reaktionsgemisch auf Eis, extrahiert mit Toluol und destilliert die organische Phase ab. Man erhält 81 g 3-(2′-Chlor-4-trifluormethyl-phenoxy)-6-chlor-thioanisol vom Sdp. 158°/0,4 mbar.

20g dieser Verbindung werden mit 30 ml 2-Brompropionsäuremethylester bei 190° 20 Stunden verrührt; dann erfolgt Destillation im Kugelrohr. Man erhält 21 g $\alpha$-[3-(2′-Chlor-4′-trifluormethyl-phenoxy)-6-chlorphenylthio]-propionsäure-methylester. Sdp. 200°/0,13 mbar.

c) 10 g dieser Verbindung werden in Methylenchlorid (200 ml) analog Beispiel 1d mit 3-Chlorperbenzoesäure umgesetzt. Die Aufarbeitung ergibt 9,5 g eines viskosen Oels mit Brechungsindex $n_D^{23}$ = 1,3295.

In analoger Weise zu deisen Beispielen wurden die folgenden Verbindungen hergestellt:

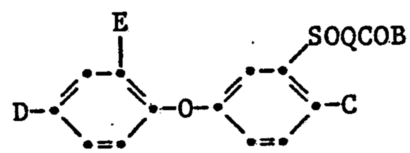

| No. | D | E | C | Q | B | phys. Daten |
|---|---|---|---|---|---|---|
| 1.1 | $CF_3$ | Cl | H | $CH(CH_3)$ | $OCH_3$ | $n_D^{23}$ 1.5500 |
| 1.2 | Cl | Cl | Cl | $CH_2$ | $OCH_3$ | $n_D^{22}$ 1.6042 |
| 1.3 | $CF_3$ | Cl | Cl | $CH_2$ | $OCH_3$ | Sdp. 220°/0.05 |
| 1.4 | Cl | Cl | I | $CH_2$ | $OCH_3$ | Smp. 120–22° |
| 1.5 | $CF_3$ | Cl | Cl | $CH(CH_3)$ | $OCH_3$ | $n_D^{23}$ 1.3295 |
| 1.6 | $CF_3$ | $NO_2$ | Cl | $CH_2$ | $OCH_3$ | $n_D^{23}$ 1.5520 |
| 1.7 | $CF_3$ | $NO_2$ | Cl | $CH_2$ | $OC_2H_4CN$ | Oel |
| 1.8 | $CF_3$ | $NO_2$ | Cl | $CH_2$ | $NH_2$ | Smp. > 200° |
| 1.9 | $CF_3$ | $NO_2$ | Cl | $CH_2$ | $NHC_2H_4OCH_3$ | Harz |

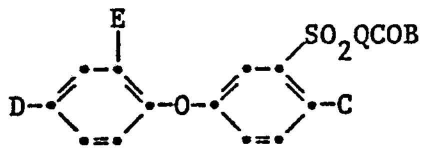

| No. | D | E | C | Q | B | phys. Daten |
|---|---|---|---|---|---|---|
| 2.1 | $CF_3$ | Cl | Cl | $CH(CH_3)$ | $OCH_3$ | $n_D^{23}$ 1.5359 |
| 2.2 | $CF_3$ | Cl | Cl | $CH(CH_3)$ | OH | Sdp. 188–95°/0.1 |
| 2.3 | $NO_2$ | Cl | Cl | $CH(CH_3)$ | $OCH_3$ | Sdp. 210°/0.1 |
| 2.4 | Cl | Cl | Cl | $CH(CH_3)$ | $OCH_3$ | $n_D^{24}$ 1.5890 |
| 2.5 | Cl | Cl | H | $CH(CH_3)$ | OH | Sdp. 215°/0.05 |
| 2.6 | Cl | Cl | H | $CH(CH_3)$ | $OCH_3$ | Sdp. 198°/0.02 |
| 2.7 | Cl | Cl | Cl | $CH_2$ | $OCH_3$ | Sdp. 210°/0.1 |
| 2.8 | Cl | Cl | Cl | $CH_2$ | OH | Sdp. 200°/0.03 |
| 2.9 | Cl | Cl | Cl | $CH_2$ | $O^{\ominus}Na^{\oplus}$ | Smp. 193° Z |
| 2.10 | Cl | Cl | Cl | $C_2H_4$ | $OCH_3$ | $n_D^{22}$ 1.5855 |
| 2.11 | $CF_3$ | Cl | $NO_2$ | $CH(CH_3)$ | $OCH_3$ | $n_D^{25}$ 1.5243 |
| 2.12 | $CF_3$ | $NO_2$ | Cl | $CH_2$ | $OCH_3$ | Sdp. 205°/0.1 |
| 2.13 | $CF_3$ | Cl | $NO_2$ | $CH(CH_3)$ | $OC_2H_4CN$ | Sdp. 195°/0.03 |
| 2.14 | $CF_3$ | Cl | $NO_2$ | $CH(CH_3)$ | $OC_2H_4OCH_3$ | Sdp. 220°/0.1 |
| 2.15 | $CF_3$ | Cl | $NO_2$ | $CH(CH_3)$ | $NH_2$ | Smp. 178° z |
| 2.16 | $CF_3$ | Cl | $NO_2$ | $CH(CH_3)$ | $N(C_2H_5)_2$ | Harz |
| 2.17 | $CF_3$ | Cl | Cl | $CH(CH_3)$ | $OCH_2-CH=CH_2$ | Sdp. 220 /0.1 |
| 2.18 | $CF_3$ | Cl | Cl | $CH(CH_3)$ | $SCH_2-CH=CH_2$ | Sdp. 190 /0.03 |
| 2.19 | $CF_3$ | Cl | Cl | $CH(CH)_3$ | 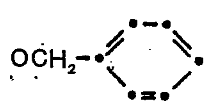 | Sdp. 212°/0.02 |
| 2.20 | $CF_3$ | Cl | Cl | $CH(CH_3)$ | $OC_2H_4Cl$ | Sdp. 230 /0.1 |

Als Zwischenprodukte benötigte Phenoxy-phenylthio-alkancarbonsäure-derivate der Formel II

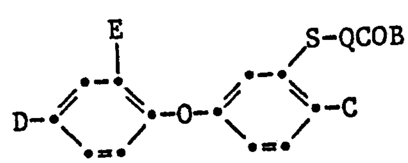

| D | E | C | Q | B | phys. Daten |
|---|---|---|---|---|---|
| Cl | Cl | CN | $CH_2$ | $OCH_3$ | Smp. 95° |
| Cl | Cl | I | $CH_2$ | $OCH_3$ | $n_D^{24}$ 1.6528 |
| Cl | Cl | Cl | $C_2H_4$ | $OCH_3$ | $n_D^{22}$ 1.6059 |
| Cl | Cl | Cl | $CH(CH_3)$ | $OCH_3$ | $n_D^{21}$ 1.6026 |
| Cl | Cl | Cl | $CH_2$ | $OCH_3$ | Sdp. 230°/0.01 |
| Cl | Cl | H | $C_2H_4$ | $OCH_3$ | $n_D^{23}$ 1.5972 |
| Cl | Cl | I | $CH(CH_3)$ | $OC_2H_5$ | $n_D^{23}$ 1.6185 |
| $CF_3$ | Cl | H | $CH(CH_3)$ | $OCH_3$ | $n_D^{23}$ 1.40°/0.08 |
| $CF_3$ | Cl | H | $C_2H_4$ | $OC_2H_5$ | Sdp. 250°/0.03 |
| $CF_3$ | $NO_2$ | Cl | $CH_2$ | $OCH_3$ | Sdp. 235°/0.5 |
| $CF_3$ | $NO_2$ | Cl | $CH(CH_3)$ | $OCH_3$ | Sdp. 235°/0.45 |
| $CF_3$ | Cl | Cl | $CH(CH_3)$ | $OCH_3$ | Sdp. 200°/0.13 |
| $CF_3$ | Cl | $OCH_3$ | $CH(CH_3)$ | $OCH_3$ | Sdp. 195°/0.04 |
| Cl | Cl | Cl | $CH(C_2H_5)$ | $OC_2H_5$ | Sdp. 182°/0.02 |
| Cl | Cl | H | $CH(C_2H_5)$ | $OC_2H_5$ | Sdp. 165°/0.001 |
| $CF_3$ | CN | Cl | $CH(CH_3)$ | $OCH_3$ | Sdp. 195°/0.001 |
| $CF_3$ | Cl | $OCH_3$ | $CH(CH_3)$ | $OC_2H_5$ | Sdp. 190°/0.001 |
| $CF_3$ | Cl | Cl | $CH_2$ | $OCH_3$ | Sdp. 190°/0.005 |
| $CF_3$ | $NO_2$ | H | $CH(C_2H_5)$ | $OCH_3$ | Sdp. 185°/0.04 |
| $CF_3$ | H | H | $CH(CH_3)$ | $OCH_3$ | $n_D^{23}$ 1.5401 |
| $CF_3$ | Cl | $NO_2$ | $CH(CH_3)$ | $OCH_3$ | Sdp. 190–95°/0.04 |
| Cl | H | H | $C_2H_4$ | $OCH_3$ | $n_D^{25}$ 1.5972 |
| $CF_3$ | $NO_2$ | $NO_2$ | $CH(CH_3)$ | $OCH_3$ | Sdp. 165°/0.001 |
| $CF_3$ | Cl | Cl | $CH(C_2H_5)$ | $OC_2H_5$ | Sdp. 155°/0.001 |

Die Phenoxy-phenylsulfinyl- und - phenylsulfonylalkan-carbonsäure-derivate der Formel I sind stabile Verbindungen, welche in üblichen organischen Lösungsmitteln, wie Alkoholen, Aethern, Ketonen, Dimethylformamid, Dimethylsulfoxyd etc. löslich sind.

Die Erfindung betrifft auch herbizide und pflanzenwachstumregulierende Mittel, welche einen neuen Wirkstoff der Formel I erhalten, sowie Verfahren zur pre- und post-emergenten Unkrautbekämpfung und zur Hemmung des Pflanzenwuchses von monocotylen und dicotylen Pflanzen, insbesondere Gräsern, Getreide, Soja und Tabakgeiztrieben.

Die erfindungsgemässen Mittel können in den üblichen Formulierungen vorliegen.

Die Herstellung erfindungsgemässer Mittel erfolgt in an sich bekannter Weise durch inniges Vermischen und Vermahlen von Wirkstoffen der Formel I mit geeigneten Trägerstoffen, gegebenenfalls unter Zusatz von gegenüber den Wirkstoffen inerten Dispersions- oder Lösungsmitteln. Die Wirkstoffe

können in den folgenden Aufarbeitungsformen vorliegen und angewendet werden:

| | |
|---|---|
| feste Aufarbeitungsformen: | Stäubemittel, Streumittel, Granulate, Umhüllungsgranulate, Imprägnierungsgranulate und Homogengranulate; |
| in Wasser dispergierbare Wirkstoffkonzentrate: | Spritzpulver, (wettable powder), Pasten, Emulsionen: |
| flüssige Aufarbeitungsformen: | Lösungen. |

Zur Herstellung fester Aufarbeitungsformen (Stäubemittel, Streumittel, Granulate) werden die Wirkstoffe mit festen Trägerstoffen vermischt. Als Trägerstoffe kommen zum Beispiel Kaolin, Talkum, Bolus, Löss, Kreide, Kalkstein, Kalkgrits, Ataclay, Dolomit, Diatomenerde, gefällte Kieselsäure, Erdalkalisilikate, Natrium- und Kalium- aluminiumsilikate (Feldspäte und Glimmer), Calcium- und Magnesiumsulfate, Magnesiumoxid, gemahlene Kunststoffe, Düngemittel, wie Ammoniumsulfat, Ammoniumphosphat, Ammoniunitrat, Harnstoff, gemahlene pflanzliche Produkte, wie Getreidemehl, Baumrindemehl, Holzmehl, Nussschalenmehl, Cellulosepulver, Rückstände von Pflanzenextraktionen, Aktivkohle, etc., je für sich oder als Mischungen untereinander in Frage.

Der Gehalt an Wirkstoff in den oben beschriebenen Mitteln liegt zwischen 0,1 bis 95%, bevorzugt zwischen 1 bis 80%. Anwendungsformen können bis hinab zu 0,001% verdünnt werden. Die Aufwandmengen betragen in der Regel 0,1 bis 10 kg AS/ha, vorzugsweise 0,25 bis 5 kg AS/ha.

Die in den erfindungsgemässen Mitteln enthaltenen Wirkstoffe beeinflussen das Pflanzenwachstum in verschiedener Weise. So hemmen, vorzögern oder unterbinden sie in erster Linie das Wachstum und die Keimung. Es handelt sich dabei also sowohl um pre- und post-emergente Herbizidwirkung als auch um Wuchshemmung.

Erfindungsgemässe Mittel, die als aktive Komponente mindestens eine Verbindung der Formel I enthalten, eignen sich besonders zur Hemmung und Kontrolle des Pflanzenwachstums von monocotylen und dicotylen Pflanzen, wie Gräsern, Sträuchern, Bäumen, Getreide- und Leguminosen-kulturen, Zuckerrohr, Tabak, Soja, Zwiebeln- und Kartoffelknollen, Zierpflanzen, Obstbäumen und Reben.

Die herbizide Wirkung von erfindungsgemässen und strukturähnlichen, bekannten Verbindungen wurde im Gewächshaus durch die folgenden Versuche geprüft.

*1. Geprüfte Verbindungen*

Die erfindungsgemässen Verbindungen sind durch die Nummer gekennzeichnet, welche sie in den obenstehenden Tabellen haben. Es wurden folgende bekannte Verbindungen geprüft:

A

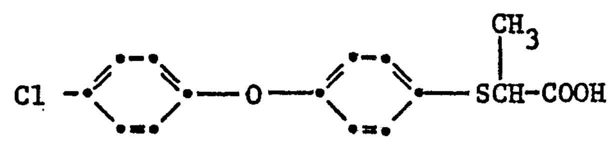

Beispiel 26 FR—PS 2 285 861 und
Verbindung 48 DOS 2 223 894

B

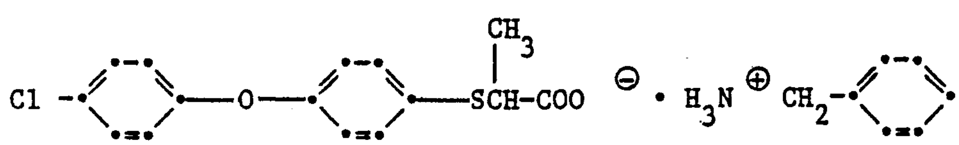

Verbindung 49 DOS 2 223 894

C

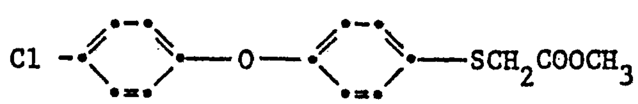

gemäss FR—PS 2 285 861 oder DOS 2 223 894

D

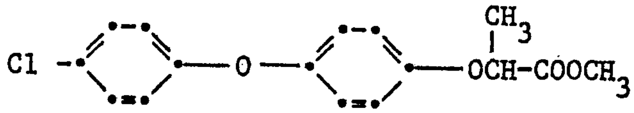

Verbindung 6 DOS 2 223 894

Diese Verbindungen wurden zu einem 25%igen Emulsionskonzentrat oben beschriebener Zusammensetzung aufgearbeitet, welches dann mit Wasser bis zu der gewünschten Wirkstoffkonzentration verdünnt wurde.

Die Prüfung der herbiziden Wirkung erfolgte wie folgt:

*Herbizide Wirkung bei Applikation der Wirkstoffe nach dem Auflaufen der Pflanzen (post-emergent).*

Verschiedene Kulturpflanzen und Unkräuter werden aus den Samen in Töpfe im Gewächshaus aufgezogen, bis sie das 4- bis 6-Blatt-Stadium erreicht haben. Dann werden die Pflanzen mit wässerigen Wirkstoffemulsionen (erhalten aus dem Emulsionskonzentrat) in verschiedenen Dosierungen gespritzt. Die behandelten Pflanzen werden dann bei optimalen Bedingungen von Licht, Begiessung, Temperatur (22—25°C) und Luftfleuchtigkeit (50—70% relativ) gehalten. Die Auswertung des Versuches erfolgte 15 Tage nach der Behandlung gemäss folgender EWRC-Wertung (European Weed Research Council):

9 = Pflanze gedeiht normal wie unbehandelte Kontrollpflanze.
8—2 linear, prozentual zunehmende Schädigung der Pflanzen
1 Pflanze abgestorben
— Pflanze nicht geprüft.

In einem ersten Durchgang wurden alle Verbindungen mit einer Aufwandmenge von 4 kg pro Hektar auf 7 Pflanzen, mono- sowie dicotyle geprüft. Die Resultate sind in der folgenden Tabelle zusammengefaast.

| Verb. No. | Hafer | Sectaria italica | Lolium perenne | Tomate | Sinapis alba | Stellaria media | Bohne |
|---|---|---|---|---|---|---|---|
| A | 7 | 1 | 2 | 7 | 6 | 7 | 9 |
| B | 8 | 8 | 7 | 7 | 6 | 5 | 7 |
| C | 7 | 7 | 8 | 8 | 6 | 5 | 7 |
| D | 1 | 1 | 1 | 1 | 1 | 1 | - |
| 1.1 | 1 | 1 | 2 | 1 | 1 | 3 | 1 |
| 1.3 | 5 | 3 | 6 | 1 | 1 | 9 | 2 |
| 1.5 | 3 | 2 | 4 | 1 | 1 | 3 | 1 |
| 2.4 | 9 | 6 | 7 | 1 | 1 | 8 | 2 |
| 2.7 | 4 | 3 | 5 | 1 | 1 | 9 | 1 |
| 2.10 | 6 | 3 | 7 | 1 | 1 | 6 | 1 |
| 2.11 | 3 | 6 | 7 | 1 | 1 | 8 | 4 |

Einige dieser Verbindungen sind einer noch eingehenderen Prüfung unterzogen worden:

| Verbindung<br>Aufwandmenge kg/ha | 1.1<br>421 1/2 | 1.5<br>421 1/2 | 2.4<br>42 | 2.10<br>42 | 2.11<br>42 | 2.12<br>42 | D<br>421 1/2 |
|---|---|---|---|---|---|---|---|
| Pflanze: | | | | | | | |
| Reis | 7889 | 7999 | 89 | 89 | 88 | 99 | 7999 |
| Hafer | 2379 | 3347 | 99 | 99 | 77 | 89 | 9999 |
| Gerste | 2267 | 1122 | 99 | 78 | 88 | 78 | 8999 |
| Weizen | 4467 | 3569 | 99 | 88 | 87 | 99 | 9999 |
| sorghum halepense | 1245 | 2336 | 78 | 37 | 44 | 78 | 1126 |
| cyperus es. | 5999 | 4459 | 78 | 34 | 33 | 66 | 8888 |
| digitaria sang. | 2234 | 2248 | 99 | 78 | 56 | 88 | 1112 |
| setaria italica | 2356 | 2445 | 89 | 67 | 44 | 88 | 1112 |
| echinochloa crus galli | 3378 | 2446 | 99 | 77 | 33 | 77 | 1111 |
| Sida spinosa | 1133 | 3367 | 68 | 22 | 33 | 68 | 3333 |
| sesbania exalt. | 1123 | 1112 | 45 | 11 | 22 | 12 | 9999 |
| amarantus retroflexus | 1111 | 1124 | 24 | 22 | 35 | 35 | 9999 |
| sinapis alba | 1223 | 1224 | 22 | 12 | 22 | 22 | 8899 |
| glaium aparine | 7877 | 1233 | 34 | 33 | 33 | 77 | 9999 |
| pastinaca sativa | 6789 | 3356 | 67 | 66 | 78 | 88 | 9999 |
| rumex sp. | 3347 | 2224 | 79 | 67 | 67 | 77 | 9999 |
| matricaria cham. | 4489 | 7888 | 88 | 45 | 23 | 88 | 9999 |
| chrysanth. leuc. | 3333 | 2222 | 33 | 22 | 12 | 25 | – |
| abutilon sp. | 1113 | 3367 | 68 | 36 | 13 | 89 | – |
| Tomate | 2223 | 3333 | 44 | 33 | 33 | 12 | – |
| Zuckerrübe | 3456 | 6778 | 66 | 44 | 77 | 77 | 6788 |
| Soja | 2334 | 6077 | 67 | 66 | 66 | 77 | 9999 |
| Baumwolle | 2334 | 1112 | 22 | 23 | 11 | 55 | 6888 |

Einige Verbindungen wurden ausserdem auf ihre selektive Herbizidwirkung in Reiskulturen geprüft.

*Selektive herbizide Wirkung auf Reis im Nachauflaufverfahren*

Reispflänzchen, welche 25 Tage als sind, wurden im Gewächshaus in grosse rechteckige Eternitschalen verpflanzt. Zwischen die Reihen der Reispflanzen wurden dann Samen der in Reiskulturen vorkommenden Unkräuter Echinochloa crus galli. Cyperus difformis, Ammania und Rotala gesät. Die Schalen wurden gut bewässert und bei einer Temperatur von ca. 25°C und hoher Luftfeuchtigkeit gehalten. Nach 12 Tagen, wenn die Unkräuter aufgelaufen sind und das 2—3 Blattstadium erreicht haben, wurde die Erde in der Schale mit einer 2,5 cm hohen Schicht Wasser bedeckt. Der Wirkstoff

wurde dann als Emulsionskonzentrat mittels einer Pipette zwischen die Pflanzenreihen appliziert, wobei man das Emulsionskonzentrat so verdünnte und auftrug, dass es einer Applikationsmenge im Feld von 4, 2, 1 und 1/2 kg Wirkstoff pro Hektar entsprach. Der Versuch wurde nach 4 Wochen ausgewertet. Die Resultate sind in der Tabelle 3 zusammengefasst.

Reisversuch

| Pflanze Aufwandmenge kg/ha | Reis 421 1/2 | echinochloa crus galli 421 1/2 | cyperus difformis 421 1/2 | ammania indica 421 1/2 | rotala indica 421 1/2 |
|---|---|---|---|---|---|
| Verbindung | | | | | |
| 1.1 | 6789 | 2578 | 4677 | 1126 | 2669 |
| 1.5 | 6788 | 1135 | 2779 | 1223 | 1224 |
| 2.10 | 99-- | 14-- | 66-- | 11-- | 13-- |
| 2.11 | 4679 | 3579 | 3569 | 1113 | 3346 |
| D | 4799 | 1112 | 5668 | 2344 | 4799 |

In diesen Versuchen zeigen die erfindungsgemässen Verbindungen generell gute Herbizidwirkung gegen dikotyle Pflanzen und einige monokotyle Unkräuter. Sie könnten beispielsweise zur selektiven Bekämpfung, vor allem zweikeimblättiger Unkräuter in Reis und gewissen Getreiden eingesetzt werden.

Die bekannten Verbindungen A, B und C zeigten hier keine ausgesprochene Herbizidwirkung, während die Verbindung D verschieden wirkt und vor allem monokotyle Unkräuter schädigt.

**Patentansprüche**

1. Phenoxy-phenyl-sulfinyl- und -sulfonyl-alkancarbonsäure-Derivate der Formel I

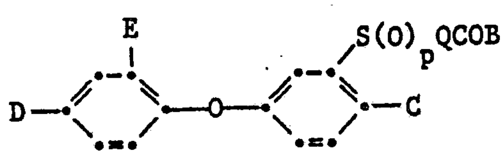

(I)

worin
B einen Rest —$OR_1$, —$SR_2$ oder —$NR_3R_4$,
C Wasserstoff, Halogen, Nitro, $C_1$—$C_4$ Alkyl oder $C_1$—$C_4$ Aloxy,
D Halogen, Cyan, Nitro oder Trifluormethyl,
E Wasserstoff, Halogen, Cyan oder Nitro,
p die Zahl 1 oder 2,
Q eine unverzweigte oder verzweigte $C_1$—$C_4$ Alkylenbrücke,
$R_1$ Wasserstoff oder das Kation eines Alkali- oder Erdalkalimetalles, einen $C_1$—$C_4$ Alkylammonio-Rest, der im Alkylteil durch Hydroxyl, Cyan oder $C_1$—$C_4$ Alkoxy substituiert sein kann,
— $C_1$—$C_{18}$ Alkyl, unsubstituiert oder substituiert durch Halogen, Nitro, Cyan, $C_1$—$C_8$ Alkoxy, Bis($C_1$—$C_4$-akyl)-amino,
— $C_3$—$C_{18}$ Alkenyl, gegebenenfalls Halogen substituiert,
— $C_3$—$C_8$ Alkinyl
— $C_3$—$C_{12}$ Cycloalkyl,
— Phenyl oder Benzyl, unsubstituiert oder substituiert durch Halogen, $C_1$—$C_4$ Alkyl, $C_1$—$C_4$ Alkoxy, $C_1$—$C_4$ Alkylthio, Nitro, Cyan oder Trifluormethyl;
$R_2$ $C_1$—$C_{18}$ Alkyl,
— Phenyl- oder Benzyl, unsubstituiert oder substituiert durch Halogen, $C_1$—$C_4$ Alkyl, $C_1$—$C_4$ Alkoxy, $C_1$—$C_4$ Alkylthio, Nitro, Cyan oder Trifluormethyl;
$R_3$ und $R_4$ Wasserstoff, $C_1$—$C_4$ Alkyl oder 2-Methoxyethyl, bedeuten.

2. Die Phenoxy-phenylsulfinyl-alkancarbonsäurederivate der Formel

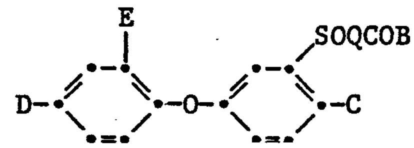

worin B, C, D, E und Q die unter Formel I, Anspruch 1 gegebene Bedeutung haben.

3. Die Phenoxy-phenylsulfonyl-alkancarbonsäurederivate der Formel

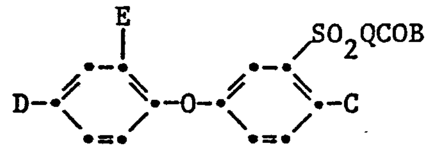

worin B, C, D, E und Q die unter Formel I, Anspruch 1 gegebene Bedeutung haben.

4. α-[3-(2′-Chlor-4′-trifluormethylphenoxy)-6-nitrophenylsulfonyl]-propionsäure-methylester.

5. α-[3-(2′,4′-Dichlorphenoxy)-6-chlor-phenylsulfonyl]-propionsäure-methylester.

6. α-[3-(2′-Nitro-4′-trifluormethylphenoxy)-6-chlor-phenoxysulfinyl]-propionsäure-methylester.

7. α-[3-(2′-Nitro-4′-trifluormethylphenoxy)-6-chlor-phenoxysulfonyl]-propionsäure-methylester.

8. Verfahren zur Herstellung der Phenoxy-phenylsulfinyl- und -sulfonyl-alkancarbonsäurederivate gemäss Anspruch 1, dadurch gekennzeichnet, dass man in an sich bekannter Weise Phenoxy-phenylthio-alkancarbonsäurederivate der Formel II

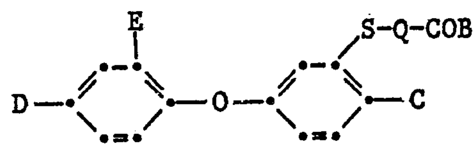

(III)

worin B, C, D, E und Q die im Anspruch 1 gegebene Bedeutung haben, solange mit einem oxydierenden Mittel behandelt, bis die Sulfid-Brücke zur Sulfinyl-(—SO—) oder Sulfonyl-Brücke (—$SO_2$—) oxydiert ist.

9. Herbizides und das Pflanzenwachstum regulierendes Mittel, dadurch gekennzeichnet, dass es als Wirkstoff mindestens ein Phenoxy-phenyl-sulfinyl- oder -sulfonylalkancarbonsäurederivate der Formel I, Anspruch 1, enthält zusammen mit in der Agrarchemie üblichen inerten Formulierungshilfsmitteln.

10. Die Verwendung der Phenoxy-phenyl-sulfinyl- oder -sulfonyl-alkancarbonsäurederivate der Formel I, Anspruch 1, oder sie enthaltender Mittel zur selektiven Bekämpfung von Unkräutern in Nutzpflanzenkulturen.

11. Die Verwendung der Phenoxy-phenyl-sulfinyl- oder -sulfonyl-alkancarbonsäurederivate der Formel I, Anspruch 1, oder sie enthaltender Mittel zur Hemmung des Pflanzenwachstums.

## Revendications

1. Dérivés d'acides phénoxy-phényl-sulfinyl- et -sulfonyl-alcane-carboxyliques de formule I

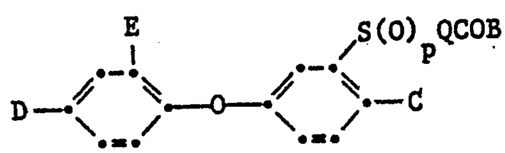

(I)

dans laquelle

B représente un reste —$OR_1$, —$SR_2$ ou —$NR_3R_4$,

C représente l'hydrogène, un halogène, un groupe nitro, alkyle en $C_1$—$C_4$ ou alcoxy en $C_1$—$C_4$,

D représente un halogène, un groupe cyano, nitro ou trifluorométhyle,

E représente l'hydrogène, un halogène, un groupe cyano ou nitro,

p est égal à 1 ou 2,

Q représente un pont alkylène droit ou ramifié en $C_1$—$C_4$,

$R_1$ représente l'hydrogène ou le cation d'un métal alcalin ou alcalino-terreux, un reste (alkyle en $C_1$—$C_4$)-ammonio qui peut être substitué dans la partie alkyle par un groupe hydroxy, cyano ou alcoxy en $C_1$—$C_4$,

— un groupe alkyle en $C_1$—$C_{18}$ non substitué ou substitué par un halogène, un groupe nitro, cyano

alcoxy en $C_1$—$C_8$, bis-(alkyle en $C_1$—$C_4$)-amino,
— un groupe alcényle en $C_3$—$C_{18}$ éventuellement substitué par un halogène,
— un groupe alcynyle en $C_3$—$C_8$,
— un groupe cycloalkyle en $C_3$—$C_{12}$,
— un groupe phényle ou benzyle non substitué ou substitué par un halogène, un groupe alkyle en $C_1$—$C_4$, alcoxy en $C_1$—$C_4$, alkylthio en $C_1$—$C_4$, nitro, cyano ou trifluorométhyle;
$R_2$ représente un groupe alkyle en $C_1$—$C_{18}$,
— un groupe phényle ou benzyle non substitué ou substitué par un halogène, un groupe alkyle en $C_1$—$C_4$, alcoxy en $C_1$—$C_4$, alkylthio en $C_1$—$C_4$, nitro, cyano ou trifluorométhyle;
$R_3$ et $R_4$ représentent l'hydrogène, un groupe alkyle en $C_1$—$C_4$ ou 2-méthoxy-éthyle.

2. Les dérivés d'acides phénoxy-phényl-sulfinyl-alcane-carboxyliques de formule

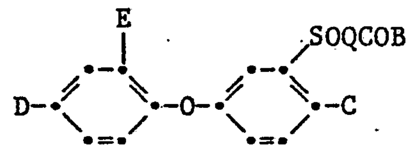

dans laquelle B, C, D, E et Q ont les significations indiquées en référence à la formule I, revendication 1.

3. Les dérivés d'acides phénoxy-phényl-sulfonyl-alcane-carboxyliques de formule

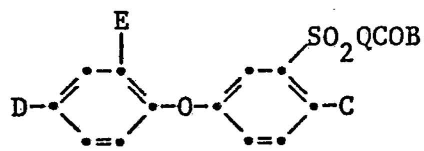

dans laquelle B, C, D, E et Q ont les significations indiquées en référence à la formule I, revenidication 1.

4. L'$\alpha$-[3-(2'-chloro-4'-trifluorométhyl-phénoxy)-6-nitrophényl-sulfonyl]-propionate de méthyle.

5. L'$\alpha$-[3-(2',4'-dichlorophénoxy)-6-chloro-phénylsulfonyl]-propionate de méthyle.

6. L'$\alpha$-[3-(2'-nitro-4'-trifluorométhyl-phénoxy)-6-chloro-phénoxy-sulfinyl]-propionate de méthyle.

7. L'$\alpha$-[3-(2'-nitro-4'-trifluorométhyl-phénoxy)-6-chloro-phénoxysulfonyl]-propionate de méthyle.

8. Procédé de préparation des dérivés d'acides phénoxy-phényl-sulfinyl- et -sulfonyl-alcane-carboxyliques selon la revendication 1, caractérisé en ce que l'on traite de manière connue en soi des dérivés d'acides phénoxy-phénylthio-alcane-carboxyliques de formule II,

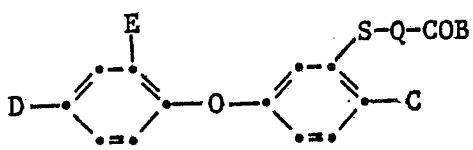

(II)

dans laquelle B, C, D, E et Q ont les significations indiquées dans la revendication 1, par un agent oxydant jusqu'à oxydation du pont sulfure en pont sulfinyle (—SO—) ou sulfonyle (—$SO_2$—).

9. Produit herbicide et régulateur de la croissance des végétaux caractérisé en ce qu'il contient en tant que substance active au moins un dérivé d'acide phénoxy-phényl-sulfinyl- ou -sulfonyl-alcane-carboxylique de formule I, revendication 1, avec des produits auxiliaires de formulation inertes usuels dans la chimie agricole.

10. L'utilisation des dérivés d'acides phénoxy-phényl-sulfinyl- ou -sulfonyl-alcane-carboxyliques de formule I, revendication 1, ou de produits en contenant, dans la lutte sélective contre les mauvaises herbes dans les cultures de végétaux utiles.

11. L'utilisation des dérivés d'acides phénoxy-phényl-sulfinyl- ou -sulfonyl-alcane-carboxyliques de formule I, revendication 1, ou de produits en contenant, pour inhiber la crossance de végétaux.

**Claims**

1. A phenoxyphenylsulfinyl- or -sulfonylalkanecarboxylic acid derivative of the formula I

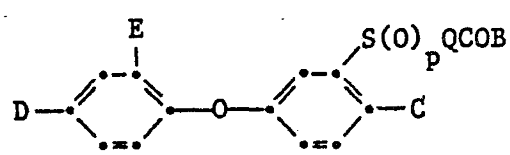

(I)

wherein
B is a radical $—OR_1$, $—SR_2$ or $—NR_3R_4$,
C is hydrogen, halogen, nitro, $C_1$—$C_4$alkyl or $C_1$—$C_4$alkoxy,
D is halogen, cyano, nitro or trifluoromethyl,
E is hydrogen, halogen, cyano or nitro,
p is 1 or 2,
Q is an unbranched or a branched $C_1$—$C_4$alkylene bridge,
$R_1$ is hydrogen or the cation of an alkali metal or alkaline earth metal; a $C_1$—$C_4$alkylammonium radical which can be substituted in the alkyl moiety by hydroxyl, cyano or $C_1$—$C_4$alkoxy; $C_1$—$C_{18}$alkyl which is unsubstituted or substituted by halogen, nitro, cyano, $C_1$—$C_8$alkoxy or bis($C_1$—$C_4$alkyl)amino; $C_3$—$C_{18}$alkenyl which is unsubstituted or substituted by halogen; $C_3$—$C_8$alkynyl; $C_3$—$C_{12}$cycloalkyl; phenyl or benzyl, unsubstituted or substituted by halogen, $C_1$—$C_4$alkyl, $C_1$—$C_4$alkoxy, $C_1$—$C_4$alkylthio, nitro, cyano or trifluoromethyl;
$R_2$ is $C_1$—$C_{18}$alkyl; phenyl or benzyl, unsubstituted or substituted by halogen, $C_1$—$C_4$alkyl, $C_1$—$C_4$alkoxy, $C_1$—$C_4$alkylthio, nitro, cyano or trifluoromethyl; and
$R_3$ and $R_4$ are $C_1$—$C_4$alkyl or 2-methoxyethyl.

2. A phenoxyphenylsulfinylalkanecarboxylic acid derivative of the formula

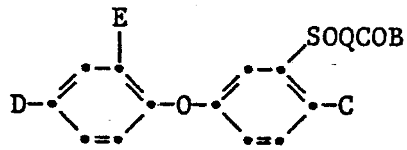

wherein B, C, D, E and Q are as defined for formula I in claim 1.

3. A phenoxyphenylsulfonylalkanecarboxylic acid derivative of the formula

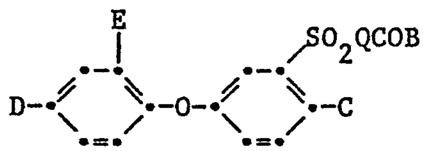

wherein B, C, D, E and Q are as defined for formula I in claim 1.

4. α-[3-(2′-Chloro-4′-trifluoromethylphenoxy)-6-nitrophenylsulfonyl]propionic acid methyl ester.

5. α-[3-(2′,4′-Dichlorophenoxy)-6-chlorophenylsulfonyl]propionic acid methyl ester.

6. α-[3-(2′-Nitro-4′-trifluoromethylphenoxy)-6-chlorophenoxysulfinyl]propionic acid methyl ester.

7. α-[3-(2′-Nitro-4′-trifluoromethylphenoxy)-6-chlorophenoxysulfonyl]propionic acid methyl ester.

8. A process for the production of a phenoxyphenyl-sulfinyl- or -sulfonylalkanecarboxylic acid derivative according to claim 1, characterised in that a phenoxy-phenylthioalkanecarboxylic acid derivative of the formula II

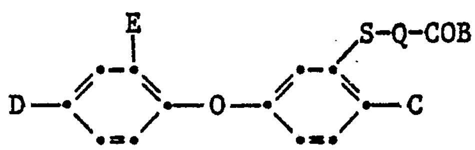

(II)

wherein B, C, D, E and Q are as defined in claim 1, is treated with an oxidising agent until the sulfide bridge is oxidised to the sulfinyl (—SO—) or sulfonyl ($—SO_2—$) bridge.

9. A herbicidal and plant growth-regulating composition, characterised in that it contains, as active ingredient, at least one phenoxyphenylsulfinyl- or -sulfonylalkanecarboxylic acid derivative of the formula I according to claim 1, together with inert formulation adjuvants conventionally employed in agricultural chemistry.

10. The use of a phenoxyphenylsulfinyl- or -sulfonylalkanecarboxylic acid derivative of the formula I according to claim 1, or of a composition containing such a compound, for selectively controlling weeds in crops of useful plants.

11. The use of a phenoxyphenylsulfinyl- or -sulfonylalkanecarboxylic acid derivative of the formula I according to claim 1, or of a composition containing such a compound, for inhibiting plant growth.